# EUROPÄISCHE PATENTANMELDUNG

(11) **EP 3 815 669 A1**
(43) Veröffentlichungstag der Anmeldung: **05.05.2021**
(21) Anmeldenummer: 19206753.6
(22) Anmeldetag: 01.11.2019
(51) Int. Cl.: A61K 8/04, A61K 8/9783, A61Q 19/00

(54) **KOSMETISCHE ZUSAMMENSETZUNG ENTHALTEND EINEN EXTRAKT AUS OPUNTIA FICUS INDICA**

(71) Anmelder: Weleda AG, 4144 Arlesheim (CH)
(72) Erfinder: HEIZLER, Daniel, 4054 Basel (CH); DA SILVA, Alissa Karyne, 4147 Aesch (BL) (CH); KAPFER, Hélène Thérèse Anne, 68300 Saint Louis (FR); HÄNNI-CIUNEL, Katarzyna, 4312 Magden (CH); IDOUX, Alicia, 4112 Bättwil (CH); SCHMITT, Meike, 79312 Emmendingen (DE)
(74) Vertreter: Simandi, Claus

(57) **Zusammenfassung**

Die Erfindung betrifft eine kosmetische Zusammensetzung enthaltend einen hydrokolloidalen Extrakt aus Opuntia ficus indica, wobei gezielt Schleimstoffe angereichert sind. Weiterhin betrifft die Erfindung einen hydrokolloidalen Extrakt aus Opuntia ficus indica und dessen Verwendung, insbesondere in der Kosmetik.

## Beschreibung

Die Erfindung betrifft eine kosmetische Zusammensetzung enthaltend einen hydrokolloidalen Extrakt aus Opuntia ficus indica, wobei gezielt Schleimstoffe angereichert sind. Weiterhin betrifft die Erfindung einen hydrokolloidalen Extrakt aus Opuntia ficus indica und dessen Verwendung.

Der Feigenkaktus (Opuntia ficus indica) ist eine Pflanzenart aus der Gattung der Opuntien (Opuntia) und gehört zur Familie der Kakteengewächse (Cactaceae). Ursprünglich stammen Opuntien, auch Feigenopuntien genannt, vom amerikanischen Kontinent, wo sowohl winterharte als auch nicht winterharte Arten zwischen Kanada und Südargentinien vorkommen und mittlerweile auch in Europa heimisch sind. Der Feigenkaktus ist nicht mit der Kaktusfeige zu verwechseln, letzterer betrifft die Frucht. Opuntia ficus indica findet vielseitige Anwendungen, wie Obstanbau, Futtermittel, Gemüse, etc.

Weleda AG forscht mit modernen und in Verbindung mit anthroposophischen Methoden an vorteilhaften natürlichen Pflanzenstoffen für die Kosmetik.

Die Haut übt als das größte Organ des Menschen zahlreiche lebenswichtige Funktionen aus. Sie schützt z.B. vor Kälte, Hitze, Strahlung, dem Einwirken chemischer Substanzen und Krankheitserregern. Sofern die Haut diese Barrierefunktion nicht mehr ausreichend erfüllt, können lokale Irritationen oder auch ganzkörperliche Beschwerden erfolgen.

Die Haut, insbesondere die Epidermis, ist als Barriereorgan des menschlichen Organismus in besonderem Maße äußeren Einwirkungen unterworfen. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedenen polaren Lipiden werden im Keratinisierungsprozess freigesetzt.

In einem optimalen Zustand der Haut liegt ein ausgewogenes Verhältnis von Hautlipiden und Hautfeuchtigkeit vor. Durch dieses Gleichgewicht werden wichtige Eigenschaften der Haut, wie das Penetrationsvermögen, Wasserbindungsvermögen, Elastizität, Regenerationsfähigkeit oder die Widerstandsfähigkeit gegen Umwelteinflüsse und Noxen unterschiedlichster Art, bestimmt. Gerade die Erhaltung der Hautfeuchtigkeit ist eine kosmetische Aufgabe zur Vorbeugung der Alterung, insbesondere Faltenbildung.

Im Stand der Technik offenbart DE 20 2016 008 641 U1 die potenzielle Eignung von Opuntia ficus indica zur Verwendung als topische Hautpflegezusammensetzung.

WO 2009/058613 offenbart eine topische Zusammensetzung, enthaltend einen Opuntia ficus indica-Extrakt (Ansprüche 5, 8 und 15), welcher die Hyaluronsäure-Produktion anregt und als Feuchtigkeitsmittel (Moisturizer) verwendet werden kann. Ferner wird beschrieben, dass der Opuntia ficus indica-Extrakt auf verschiedene Weise erhalten werden kann.

Weiterhin ist die kosmetische Eignung von Kaktusfeigenkernöl in EP 3 102 181 B1 beschrieben.

Die besondere Rolle der natürlichen Schleimstoffe von Opuntia ficus indica in der Kosmetik, welche vorzugsweise aus Pflanzenteilen bzw. Pflanzenzellen, jedoch vorzugsweise aus den Kladodien gewonnen werden können, werden jedoch im Stand der Technik nicht erkannt.

Erfindungsgemäß sind Schleimstoffe aus Opuntia ficus indica, solche die mit höchstem Anteil aus unterschiedlich aufgebauten hochmolekularen Polysacchariden bestehen, und anteilig Polyphenole, Phenole und andere sekundäre Pflanzeninhaltsstoffe enthalten können. Ihre komplexen, hochmolekularen Strukturen sind erfindungsgemäß besonders geeignet hohe Mengen an Wasser aufzunehmen, so dass schleimartige Kolloide und Gele bzw. Hydrokolloide ausgebildet werden können. Darüber hinaus wirken diese Schleimstoffe auf den Organismus vor allem reizmildernd, schleimhautschützend und entzündungshemmend.

Der vorliegenden Erfindung liegt daher die Aufgabe zugrunde, eine kosmetische Zusammensetzung zur topischen Verwendung aus Schleimstoffen von Opuntia ficus indica bereitzustellen als auch ein Verfahren zur Herstellung eines hydrokolloidalen Extrakts aus Opuntia ficus indica, wobei die Schleimstoffe aus Opuntia ficus indica angereichert werden können.

Überraschender Weise konnten die Erfinder feststellen, dass der reine hydrokolloidale Extrakt mit angereicherten Anteilen an Schleimstoffen eine besonders vorteilhafte kosmetische Wirkung entfaltet und auf natürliche Weise intensiv und nachhaltig zur Hautbefeuchtung beiträgt.

Die Aufgabe wird durch ein Verfahren zur Herstellung eines hydrokolloidalen Extraktes aus Pflanzenmaterial von Opuntia ficus indica gelöst, wobei
a.) aus Pflanzenmaterial von Opuntia ficus indica ein wässriger oder vorzugsweise wässrig-alkoholischer Auszug mit maximal 40 % (m/m) Alkohol hergestellt wird, wobei der Alkohol ein C1-C4 oder C1-C3 Alkohol sein kann, jedoch vorzugsweise Ethanol (C2) ist,
b.) der erhaltene Auszug in der Wärme bei 60 -90 Grad Celsius für eine Stunde oder länger behandelt wird,
c.) nach Abkühlung mazeriert und gefiltert wird.

In einer bevorzugten Ausführungsform kann die Mazeration mindestens über 7 Stunden als auch über Tage erfolgen, bevor die Filtration erfolgt. Weiterhin kann nach Filtration eine erneute Mazeration erfolgen. Die Mazeration kann ebenfalls als Bewegungsmazeration erfolgen, wobei die Bewegung vorzugsweise mit Hilfe von Rühren bewirkt wird.

Die Filtration gemäß Schritt c.) erfolgt vorzugsweise mit einem oder mehrere Sieben, wobei die Sieböffnungen vorzugsweise 60-300 µm, insbesondere 80-250 µm oder 120 - 200 µm betragen. Dies erlaubt das Zurückhalten von Feststoffen, so dass ein reiner und stabiler hydrokolloidaler Extrakt aus Opuntia ficus indica erhalten werden kann.

Schritt c.) kann einmal oder mehrmals wiederholt werden, ggfs. so lange bis der hydrokolloidale Extrakt vollständig ausgebildet ist, und sämtliche Schleimstoffe in den Extrakt erschöpfend überführt sind.

In einer besonders bevorzugten Ausführungsform beträgt der Anteil an Alkohol, insbesondere Ethanol max. 35 % (m/m) (35:65) im alkoholischen-wässrigen, insbesondere ethanolischwässrigen Auszug, vorzugsweise 30 % (30:70) oder gar 20 % (20 : 80) und weniger, jedoch vorzugsweise mehr als 10 %. Weiterhin können C1-C4 oder C1-C3 Alkohole eingesetzt werden, wie Methanol (C1), Propanol (C3), Butanol (C4), vorzugsweise jedoch Ethanol (C2).

Die Erfinder konnten feststellen, dass bereits ab 30 % Alkohol/Ethanol die Schleimstoffe nachteilig ausfallen und nicht in den hydrokolloidalen Extrakt überführt werden können. Weiterhin ist bevorzugt, dass das eingesetzte Ethanol 94.00 % m/m Ethanol enthält. Weiterhin ist eine rein wässrige Extraktion zwar möglich, jedoch ist die vorgenannte alkoholisch-wässrige Extraktion erfindungsgemäß bevorzugt, so dass die Schleimstoffe in den hydrokolloidalen Extrakt zeitlich effizient vollständig überführt werden können und insbesondere solche Anteile an Schleimstoffen überführt werden können, welche sekundäre Pflanzeninhaltsstoffe (Polyphenole, Phenole, Flavonoide u.a.) auch in Verbindung mit Polysacchariden aufweisen. Zudem ist nachteilig, dass der wässrige Auszug nicht im Nachhinein z.B. mit Alkohol zur Konservierung belastet werden kann, da die Schleimstoffe ausfallen.

Bei der Mazeration gemäß Schritt c.) wird das Pflanzenmaterial eine bestimmte Zeit in eine Flüssigkeit, insbesondere in der bestehenden Flüssigkeit, jedoch vorzugsweise in eine wässrigethanolische Flüssigkeit (supra) eingelegt, wobei das Mazerat (Flüssigkeit) den hydrokolloidalen Extrakt umfasst.

In einer bevorzugten Ausführungsform wird zu Schritt a.) das Pflanzenmaterial getrocknet und zerkleinert. Blüten sind vorzugsweise nicht als Pflanzenmaterial zu berücksichtigen und ggfs. auszunehmen. Besonders bevorzugt ist jedoch die Verwendung von Kladodien aus Opuntia ficus indica.

In einer weiter bevorzugten Ausführungsform kann die Wärmebehandlung gemäß Schritt b) unter Rühren erfolgen.

Weiterhin ist bevorzugt, dass die Wärmebehandlung gemäß Schritt b) bei 65 - 85 Grad Celsius, insbesondere bei 70 bis 80 Grad Celsius, insbesondere 80 Grad Celsius erfolgt.

Weiterhin ist bevorzugt, dass die Wärmebehandlung gemäß Schritt b) innerhalb 1 h erfolgt.

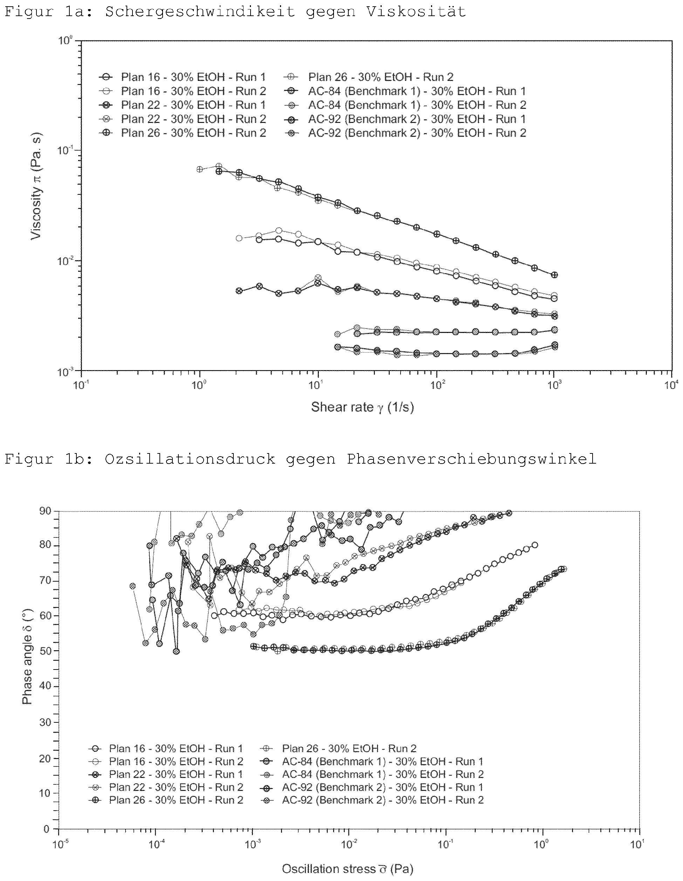

Der erfindungsgemäße hydrokolloidale Extrakt mit angereichertem Anteil an Schleimstoffen zeigt ein vorteilhaftes nicht-newtonsches Fliessverhalten in Abhängigkeit der Schergeschwindigkeit zur Viskosität als auch Oszillationsmessungen, siehe Beispiele und Figur 1a und 1b.

Dieses rheologische Verhalten ist eindeutig auf die hydrokolloidalen Eigenschaften des schleimstoffreichen Extrakts zurück zu führen.

Der erfindungsgemäße Extrakt weist durch den erhöhten Schleimstoffgehalt das oben erläuterte nicht-newtonsche Fliessverhalten auf. Die rheologische Eigenschaften können als Beleg, ein Differenzierungs- und Qualitätsmerkmal für die besondere Eignung als Feuchtigkeitsmittel verwendet zu werden, da der hydrokolloidale Charakter des Extrakts für sein wasserbindendes Vermögen verantwortlich ist.

Das erfindungsgemäße Verfahren erlaubt die vorteilhafte Anreicherung von Schleimstoffen, welche im Wesentlichen die eigenen nativ in Opuntia ficus indica enthaltenen Polysaccharide umfassen. Es handelt sich hierbei um Extrakteigene Polysaccharide, die aufgrund des erfindungsgemäßen Verfahrens aus dem Pflanzenstoff ausgelöst und als Schleimstoff angereichert werden, jedoch nicht zugesetzt werden müssen.

Daher betrifft die Erfindung ebenfalls einen Opuntia ficus indica-Extrakt, insbesondere einen hydrokolloidalen Extrakt erhältlich aus dem erfindungsgemäßen Verfahren.

Ein erfindungsgemäßes Kosmetikum weist daher keine zusätzlichen Zucker auf, wie z.B. Maltodextrin o.a., sondern verfügt über einen hydrokolloidalen Extrakt mit einzigartigen Schleimstoffen enthaltend insbesondere Polysaccharide mit hervorragenden Eigenschaften zur Aufrechterhaltung der Hautfeuchtigkeit (Moisturizing). Folglich wird erfindungsgemäß ein hervorragendes hautfeuchtigkeitsbindendes und -erhaltendes Mittel erhalten. In allen Altersgruppen ist die Versorgung der Haut mit Feuchtigkeit wesentlich. Daher ist es besonders vorteilhaft, wenn Stoffe und Zubereitungen eine hohe Feuchtigkeitsanreicherung aufweisen. Reduzierte Hautfeuchtigkeit führt zu unerwünschten Hautzuständen, die u.a. den Alterungsprozess beschleunigen können.

In einer weiteren Ausführungsform kann die erfindungsgemäße kosmetische oder dermatologische Zubereitung zur topischen Verwendung in Form eines Gels, einer Creme oder einer Lotion erfolgen, wobei vorzugsweise wässrige Systeme verwendet werden.

Emulgatoren können vorteilhaft gewählt werden aus der Gruppe der nichtionischen, anionischen, kationischen oder amphoteren Emulgatoren.

Erfindungsgemäß bevorzugt sind natürlich vorkommende Emulgatoren, zu denen beispielsweise Bienenwachs, Wollwachs, Lecithin und Sterole u.a. gehören, die ebenfalls bei der Herstellung einer erfindungsgemäßen Zusammensetzung eingesetzt werden können.

Die erfindungsgemäßen kosmetischen Zusammensetzungen können weiterhin kosmetische Hilfsstoffe enthalten, wie sie üblicherweise in solchen Zubereitungen verwendet werden, z. B. Antioxidantien, Radikalfänger, Konservierungsmittel, Bakterizide, Parfüme, Farbstoffe, Pigmente, die eine färbende Wirkung haben, Verdickungsmittel, oberflächenaktive Substanzen, weichmachende, oder andere übliche Bestandteile einer kosmetischen Formulierung wie Alkohole, Polyole, Polymere, Schaumstabilisatoren, Elektrolyte, organische Lösemittel.

In einer weiteren besonderen Ausführungsform besteht die erfindungsgemäße Zusammensetzung vorzugsweise aus natürlich vorkommenden oder naturnahen, resp. naturidentischen Inhaltsstoffen.

Nachfolgende Ausführungsbeispiele und Figuren dienen zur Erläuterung der Erfindung, ohne die Erfindung auf diese Beispiele zu beschränken.

### Beispiel 1:

Die feuchtigkeitsspendenden Eigenschaften des Extraktes aus Opuntia ficus indica Kladodien wurden in einem Vergleichstest mit kommerziell erhältlichen Extrakten (Benchmarks) in einer in-vivo Studie an 29 Probanden durchgeführt. Die jeweiligen Extrakte wurden in eine kosmetische Modellrezeptur eingearbeitet, die aufgrund ihrer Zusammensetzung keine feuchtigkeitsspendenden Eigenschaften aufweist und als Placebo verwendet wurde.

| **Inhaltsstoff (INCI)** | **Gewichtsanteil** |
|---|---|
| Aqua | **ad 100%** |
| Glyceryl stearate citrate | **7.00%** |
| Caprylic/Capric triglyceride | **6.00%** |
| Octyldodecanol | **4.00%** |
| Cetyl alcohol | **0.86%** |
| Benzyl Alcohol | **0.10%** |
| Benzoic Acid | **0.10%** |
| Sorbic acid | **0.05%** |
| Citric acid | **0.05%** |

Die feuchtigkeitsspendenden Eigenschaften der Testrezepturen wurden mit einem Corneometer (Corneometer CM® 825 (Courage & Khazaka electronic) mit Hilfe der kapazitiven Bestimmung des Wechselstromwiderstandes bestimmt. Die ermittelten Werte korrelieren mit der Hautfeuchte der oberen Hautschichten. Die Signifikanz der Resultate wurde mittels p-Wert evaluiert. Die Modellrezeptur versetzt mit 2% des schleimstoffangereicherten Opuntia ficus indica Extrakts verglichen mit der gleichen Konzentration von zwei kommerziell erhältlichen Opuntia ficus indica Extrakten: Benchmark 1 und Benchmark 2, zeigt appliziert für 2h, 4h und 24h auf der Vorderarmhaut des Probanden einen erheblich höheren durchschnittlichen Anstieg in der Hydratation der Hautschichten (siehe Tabelle):

| | Anstieg in Hydratation | | |
|---|---|---|---|
| | nach 2h | nach 4h | nach 24h |
| Extrakt mit angereichertem Schleimstoffanteil | 13% | 14% | 8% |
| Benchmark 1 | 5% | 5% | nicht signifikant |
| Benchmark 2 | 6% | 5% | -4% |

Die Resultate zeigen, dass die kosmetische Modellrezeptur einen signifikanten Anstieg in der Hydratation nach 2h, 4h und 24h aufweist, wenn sie mit 2% des schleimstoffangereicherten Opuntia ficus indica Kladodien Extrakt versetzt wird. Kommerziell verfügbare Benchmarks zeigen bei gleicher Einsatzkonzentration deutlich reduzierten oder einen nicht signifikanten oder sogar einen negativen Effekt.

### Beispiel 2:

### Viskositäts- und Schergeschwindigkeitsmessung mit einem Rheometer (DHR2, TA Instruments)

Die Figur 1a zeigt anhand der erfindungsgemäß hergestellten Extrakte, bezeichnet als Plan 16, Plan 22 und Plan 26 im Vergleich zu den kommerziell erhältlichen Benchmark-Extrakten aus Opuntia ficus indica Kladodien (Muster AC-84 und AC-92), ein Verhalten eines nicht-newtonschen Fluids, während Muster AC-84 und AC-92 ein newtonsches Fluidverhalten zeigen.

**Tabelle 1: Viskosität vs. Schergeschwindigkeit**

| **T** | **Viskosität (mPa.s) at 10E2 s-1** | | | **Viskosität (mPa.s) at 10E3 s-1** | | |
|---|---|---|---|---|---|---|
| **Probe** | **Durchlauf 1** | **Durchlauf 2** | **Mittelwert** | **Durchlauf1** | **Durchlauf 2** | **Mittelwert** |
| AC-84 | 2.24 | 2.26 | 2.25 | 2.34 | 2.36 | 2.35 |
| AC-92 | 1.45 | 1.40 | 1.43 | 1.71 | 1.66 | 1.68 |
| Plan 16 | 8.10 | 8.67 | **8.38** | 4.44 | 4.78 | **4.61** |
| Plan 22 | 4.51 | 4.61 | **4.56** | 3.19 | 3.28 | **3.24** |
| Plan 26 | 17.6 | 17.4 | **17.5** | 7.50 | 7.40 | **7.45** |

Figur 1b zeigt mittels einer Oszillationsmesstechnik, dass der Phasenverschiebungswinkel in den schleimstoffreichen hydrokolloidalen Extrakten (Muster: Plan 16, Plan 22 und Plan 26) im breiten Oszillationsbereich reduziert und konstant bei 45°-65° liegt, welches auf die tendenziell elastischen und strukturierten hydrokolloidalen Netzwerkstrukturen hinweist. Die rheologischen Eigenschaften, insbesondere das nicht-newtonsche Fliessverhalten unterscheidet sich deutlich von den kommerziell erhältlichen Opuntia ficus indica - Extrakten (Muster AC-84 und AC-92). Muster AC-84 und AC-92 zeigen bei den ausgesetzten Oszillationen eine wesentlich niedrigere Null-Scherviskosität.

**Tabelle 2:**

| **Null-Scherviskosität (mPa.s)** | | | |
|---|---|---|---|
| Probe | **Durchlauf 1** | **Durchlauf 2** | **Mittelwert** |
| AC-84* | 2.23 | 2.53 | 2.38 |
| AC-92* | 1.52 | 1.74 | 1.63 |
| Plan 16 | 25.4 | 27.5 | 26.4 |
| Plan 22 | 5.97 | 5.55 | 5.76 |
| Plan 26 | 73.4 | 104 | 88.9 |

## Patentansprüche

1. Verfahren zur Herstellung eines hydrokolloidalen Extraktes aus Opuntia ficus indica, **dadurch gekennzeichnet, dass**
a.) aus Pflanzenmaterial von Opuntia ficus indica ein wässriger oder wässrig-alkoholischer Auszug mit maximal 40 % (m/m) Alkohol hergestellt wird,
b.) der erhaltene Auszug in der Wärme bei 60 -90 Grad Celsius für eine Stunde oder länger behandelt wird,
c.) nach Abkühlung mazeriert und gefiltert wird.

2. Verfahren zur Herstellung eines hydrokolloidalen Extraktes aus Opuntia ficus indica nach Anspruch 1, wobei der Alkohol ein C1-C4 oder C1-C3 Alkohol ist, vorzugsweise Ethanol (C2).

3. Verfahren zur Herstellung eines hydrokolloidalen Extraktes aus Opuntia ficus indica nach Anspruch 1 oder Anspruch 2, wobei der wässrig-alkoholische Auszug mit maximal 30 % (m/m) Alkohol oder 20 % (m/m) Alkohol, insbesondere Ethanol hergestellt wird.

4. Verfahren zur Herstellung eines hydrokolloidalen Extraktes aus Opuntia ficus indica nach einem der vorhergehenden Ansprüche 1 bis 3, **dadurch gekennzeichnet, dass** das Filtern in Schritt c.) mit ein oder mehreren Sieben erfolgt, wobei die Sieböffnungen 60-300 µm betragen.

5. Hydrokolloidaler Extrakt aus Opuntia ficus indica erhältlich nach einem Verfahren gemäß einem der Ansprüche 1 bis 4.

6. Kosmetikum enthaltend einen hydrokolloidalen Extrakt nach Anspruch 5.

7. Feuchtigkeitsspendendes Mittel enthaltend einen hydrokolloidalen Extrakt nach einem der Ansprüche 5 - 6.
